(19) Europäisches Patentamt
European Patent Office
Office européen des brevets

(11) Veröffentlichungsnummer: **0 136 543**
**B1**

# (12) EUROPÄISCHE PATENTSCHRIFT

(45) Veröffentlichungstag der Patentschrift: **23.11.88**

(51) Int. Cl.⁴: **C 07 H 21/00**, C 07 H 19/04 // C07F9/48

(21) Anmeldenummer: **84110314.6**

(22) Anmeldetag: **30.08.84**

(54) **Verfahren zur Herstellung von Nukleosidalkyl-, -aralkyl- und -arylphosphoniten und -phosphonaten.**

(30) Priorität: **06.09.83 DE 3332068**

(43) Veröffentlichungstag der Anmeldung:
**10.04.85 Patentblatt 85/15**

(45) Bekanntmachung des Hinweises auf die Patenterteilung:
**23.11.88 Patentblatt 88/47**

(84) Benannte Vertragsstaaten:
**AT BE CH DE FR GB IT LI LU NL SE**

(56) Entgegenhaltungen:
**EP-A-0 040 099**
**EP-A-0 061 746**
**EP-A-0 090 789**
**DE-A-3 239 888**

**TETRAHEDRON LETTERS, Band 24, Nr. 9, 1983, Seiten 877-880, Pergamon Press Ltd., Oxford, GB; N.D. SINHA et al.: "A new synthesis of oligodeoxynucleoside methylphosphonates on control pore glass polymer support using phosphite approach"**
**ANGEWANDTE CHEMIE SUPPLEMENT, 1982, Seiten 2010-2015, Verlag Chemie GmbH, Weiheim, DE; J. ENGELS et al.: "Eine neue Synthese von Nukleosidmethylphosphonaten"**
**TETRAHEDRON LETTERS, Band 25, Nr. 14, 1984, Seiten 1437-1440, Pergamon Press Ltd., Oxford, GB; A. JÄGER et al.: "Synthesis of deoxynucleoside**

(73) Patentinhaber: **HOECHST AKTIENGESELLSCHAFT, Postfach 80 03 20, D-6230 Frankfurt am Main 80 (DE)**

(72) Erfinder: **Engels, Joachim, Dr., Feldbergstrasse 1, D-6242 Kronberg/Taunus (DE)**
Erfinder: **Jäger, Alfred, Dr., 1904 Jefferson Park Avenue, Nr. 46, Charlottesville Virginia 22 903 (US)**

(56) Entgegenhaltungen: (Fortsetzung)
**methylphosphonates via a phosphonamidite approach"**
**TETRAHEDRON LETTERS, Band 40, Nr. 1, 1984, Seiten 95-102, Pergamon Press Ltd., Oxford, GB; M.A. DORMAN et al.: "Synthesis of oligodeoxynucleotides and oligodeoxynucleotide analogs using phosphoramidite intermediates"**
**Methoden der organischen Chemie (Houben-Weyl), Band XII/1, S. 324-8 und 334-6 und Band E 1, S. 285-6 und 294-6**

EP 0 136 543 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Nichtionische Analoga von Desoxyribonukleinsäuren (DNA) sind von Bedeutung zur Untersuchung von DNA-DNA- und DNA-Protein-Wechselwirkungen. Besonders Phosphonsäureester der Desoxyribonukleotide sind infolge ihrer chemischen Stabilität, aufgrund ihrer Zellgängigkeit sowie ihrer erhöhten Resistenz gegen zelluläre Nukleasen von Interesse. Bisher sind vier verschiedene Synthesestrategien zur Synthese von Methylphosphonatanaloga der Nukleotide beschrieben:

1. Ogilvie et al. (M.J. Nemer und K.K. Ogilvie, Tetrahedron Lett. 21, Seite 4149 (1980)) stellten ein vollständig geschütztes Uridyl-3',5'-uridin-methylphosphonat durch Michaelis-Arbuzovsche Umlagerung der entsprechenden Phosphitzwischenstufe her. Diese Reaktion (Methyljodid, 20 Stunden bei 50°C) dürfte infolge ihrer drastischen Bedingungen nicht allgemein anwendbar sein, da z. B. eine Methylierung der Purinbasen zu erwarten ist.
2. Ts'o et al. (P.S. Miller, J. Yano, E. Yano, C. Caroll, K. Jayaraman und P.O.P. Ts'o, Biochemistry 18, 5134 (1979); Proc. Natl. Acad. Sci. USA 78, 1537 (1981); P.S. Miller, N. Drean, S.M. Pulford, K.B. Mcparland, J. Biol.Chem. 225, 9659 (1980)) entwickelten eine Synthesestrategie, die analog zur Phosphotriestermethode in der Oligonukleotidsynthese ist. Dabei wird als wichtigste Zwischenstufe ein geschützter Nukleotid-3'-0-methylphosphonsäure-β-cyanoethylester verwendet. Diese Methode besitzt die bekannten Vor- und Nachteile der Phosphotriestermethode, wobei speziell als Nachteil die geringe Reaktivität der Phosphor(V)-Verbindung zu erwähnen ist.
3. Agarwal et al. (K.L. Agarwal und F. Riftina, Nucl. Acid Res. 6, 3009 (1979)) verwendeten Methylphosphonsäuredichlorid als bifunktionelles Phosphonylierungsmittel. Beim zweiten Schritt bedarf es einer Aktivierung des Chlorids durch Tetrazol. Das erhaltene Rohprodukt ist nur über eine effiziente Chromatographie zu reinigen.
4. Ausgegangen von Methyldichlorphosphan sind J. Engels und A. Jäger, Angew. Chem. Suppl. 1982, 2010. Dieses Ausgangsmaterial führt jedoch zur Bildung von 8 Prozent symmetrischer 3',3'-Verbindung als Nebenprodukt. Es ist auch angegeben, daß die Phosphonigsäureesterverbindung glatt mit Schwefel und Selen reagiert und so den Zugang zu den thio- und selenophosphonatanalogen Verbindungen eröffnet. N.D. Sinha V. Grossbruchhaus und H. Köster, Tetrahedron Lett. 24, 877 (1983) synthetisierten die Nukleotidmethylphosphonate am polymeren Träger. Eine Charakterisierung der erhaltenen Produkte steht noch aus.
5. Die Herstellung von Phosphonigsäurediestern aus Phosphonigsäureesteramiden und deren Herstellung aus Phosphonigsäureamidhalogeniden ist an sich bekannt (Houben-Weyl, Methoden der organischen Chemie XII/I, 324-328, 334-336 (1963); EI, 285-286, 294-296 (1982)). Für die Bildung der Esteramide wird hierbei eine Durchschnittsausbeute von 60 bis 75 % und für die Bildung der Diester von 50 bis 70 % angegeben. Nukleotide als Alkoholkomponenten sind dort jedoch nicht genannt.
6. Aus der EP-AI 0 090 789 ist es bekannt, Oligonukleotide dadurch herzustellen, daß man ein an einen festen Träger gebundenes Nukleosid oder Oligonukleotid mit einem sterisch gehinderten Dialkylaminonukleosidphosphit kondensiert. Bei diesen Phosphoramid-Zwischenprodukten sind also die beiden anderen organischen Reste über Sauerstoff an den dreibindigen Phosphor gebunden.

Während bei dem Methylphosphonsäuredichlorid die Reaktivität des zweiten Halogens meist zu gering ist und einer zusätzlichen Aktivierung bedarf, ist im Falle der Phosphinsäuredichloride eher die Aktivität zu hoch. So ergeben sich Schwierigkeiten bei der Handhabung (extrem wasserfreies Medium) und obendrein wird unausweichlich symmetrischer Phosphonigsäureester gebildet.

Die Erfindung betrifft demgegenüber ein Verfahren zur Herstellung von Desoxyribonukleosidphosphonaten der allgemeinen Formel I

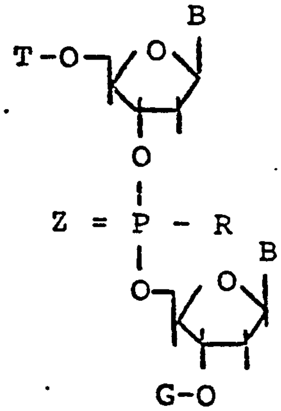

(I)

in der

T eine Schutzgruppe für eine primäre Hydroxygruppe, vorzugsweise Triphenylmethyl (= Tr), p-Anisoyldiphenylmethyl oder Di-(p-anisoyl)-phenylmethyl,
B ein Nucleosidbasenrest, bei dem eine vorhandene Exo-Aminofunktion geschützt ist, vorzugsweise 1-Thyminyl, 1-(N-4-Benzoylcytosinyl), 9-(N-6-Benzoyladeninyl) oder 9-(N-2-Isobutyroylguaninyl),
G eine Schutzgruppe für eine sekundäre Hydroxygruppe,
Z Sauerstoff, Schwefel oder Selen und
R Alkyl mit bis zu 8 C-Atomen, Cyclohexyl, Benzyl oder gegebenenfalls durch Fluor, Chlor, Brom, niederes Alkyl, niederes Alkoxy oder Trifluormethyl substituiertes Phenyl, vorzugsweise Methyl, Ethyl, Phenyl oder Benzyl, insbesondere Methyl, bedeuten,

das dadurch gekennzeichnet ist, daß man ein bifunktionelles Phosphonylierungsreagenz der allgemeinen Formel II

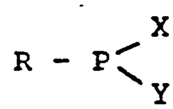 (II)

worin X Chlor oder Y und Y eine Gruppe der Formel

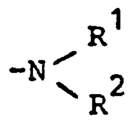

bedeuten, wobei $R^1$ und $R^2$ für gleiche oder verschiedene Alkyl- oder Cycloalkylreste mit bis zu 8 C-Atomen oder Phenylreste stehen oder $R^1$ und $R^2$ zusammen mit dem Stickstoff einen gesättigten oder ungesättigten heterocyclischen Ring, der weitere Heteroatome enthalten kann, darstellen, mit einem Nucleosid der allgemeinen Formel III

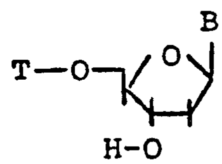 (III)

worin T und B die oben angegebene Bedeutung haben, umsetzt, vorzugsweise bei -80 bis +100°C, insbesondere bei -20 bis 0°C, und die erhaltene Verbindung der allgemeinen Formel IV

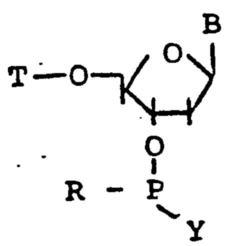 (IV)

mit einer Verbindung der allgemeinen Formel V

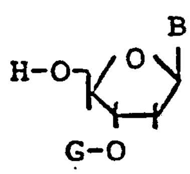 (V)

worin B und G die oben angegebene Bedeutung haben, vorzugsweise bei -20 bis +100°C, insbesondere bei Raumtemperatur umsetzt und die erhaltene Verbindung der allgemeinen Formel VI

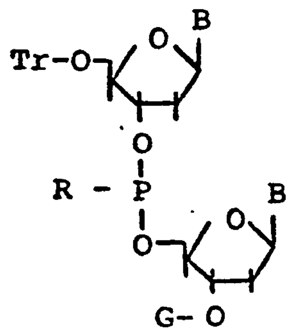

(VI)

worin T, R, B und G die oben angegebene Bedeutung haben, oxidativ in Verbindungen der allgemeinen Formel I überführt, vorzugsweise bei -80 bis +100° C, insbesondere bei -20° C-Raumtemperatur.

Die Zwischenprodukte der allgemeinen Formel IV sind neu und ebenfalls Gegenstand der Erfindung.

Der Rest R an dem bifunktionellen Phosphonylierungsreagenz der allgemeinen Formel II kann grundsätzlich jeder organische, zellungiftige Rest sein, der inert ist gegenüber den Verbindungen der allgemeinen Formeln II bis VI und der die Reaktionen nicht behindert.

Als Gruppe der allgemeinen Formel $-NR^1R^2$ kommen beispielsweise in Frage:

Dimethylamino, Diethylamino, Diisopropylamino, Methylethylamino, Methylpropylamino, Methylhexylamino, Methylcyclohexylamino, Methylbenzylamino, Morpholino, Pyrrolidino, Piperidino, Methylanilino, Diphenylamino, Imidazolo, Triazolo, Benzotriazolo und Tetrazolo.

Die Ausgangsmaterialien der allgemeinen Formel II, in denen X Chlor bedeutet, sind erhältlich durch Umsetzung des entsprechenden Dichlorphosphans, vorzugsweise Methyldichlorphosphan, mit einem sekundären Amin der allgemeinen Formel VII

$$H - NR^1R^2 \qquad \text{(VII)}$$

in der $R^1$ und $R^2$ die oben genannten Bedeutungen haben. Verbindungen der allgemeinen Formel II, in der X eine Gruppe der Formel Y bedeutet, sind entsprechend durch weitere Umsetzung mit demselben oder einem anderen sekundären Amin der allgemeinen Formel VII zugänglich. Die Verbindungen der Formel II lassen sich durch Vakuumdestillation reinigen.

Die Umsetzung des Phosphonylierungsreagenzes der allgemeinen Formel II mit einem geeignet geschützten Nukleosid der allgemeinen Formel III erfolgt in einem mäßig polaren Lösungsmittel, bevorzugt Chloroform, unter Feuchtigkeitsausschluß. Als Hilfsbase für diese Reaktion können tertiäre Amine, bevorzugt Ethyldiisopropylamin (Hünig-Base), verwendet werden. Die Aufarbeitung erfolgt durch eine wäßrige Extraktion und Fällen der Produkte der allgemeinen Formel IV mit einem unpolaren Lösemittel wie Petrolether oder Pentan. Die so gewonnenen Phosphonigsäureesteramide der allgemeinen Formel IV fallen als farblose Pulver aus und sind durch spektroskopische Daten wie $^1H$-NMR, $^{31}P$-NMR oder UV und Elementaranalyse charakterisierbar. Ferner lassen sie sich auch durch direkte Oxidation in die Phosphonsäureesteramide der allgemeinen Formel VIII

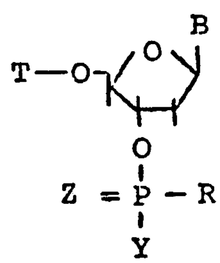

(VIII)

wobei T, B, Z, R und Y die oben angegebene Bedeutung haben, überführen, die dann isoliert und charakterisiert werden können.

Hervorzuheben ist, daß innerhalb der Nachweisgrenze kein symmetrisches 3',3'-Dinucleosidphosphonit gebildet wird.

Die Verbindungen der allgemeinen Formel I sind als Pulver, wenn trocken und mindestens bei -20° C aufbewahrt, mindestens 1 Monat stabil, wie das $^{31}P$-NMR zeigt. Diese große Beständigkeit der Phosphonigsäureesteramide ist erstaunlich und unterstreicht den Wert dieser Methode. Ihre universelle Verwendbarkeit im Aufbau von Phosphonsäurediestern der Nukleoside zeigt sich bei der Umsetzung mit geeignet 3'-geschützten Nukleosiden:

Hierbei werden die 5'-geschützten Nukleosidphosphonite der allgemeinen Formel IV in einem mäßig polaren

Lösemittel, bevorzugt Acetonitril, Chloroform oder Tetrahydrofuran, gelöst und mit dem Nukleosid der allgemeinen Formel V (in 3'-Stellung geschützt) vermischt. Geeignete Schutzgruppen G an den Verbindungen der allgemeinen Formel V sind Acylgruppen wie Benzoyl, Acetyl, Pivaloyl oder Laevulonyl oder Silylgruppen wie t-Butyl-dimethylsilyl. Die Reaktion wird durch eine Säure, vorzugsweise ein Azol oder Aminhydrochlorid, katalysiert. Besonders geeignet ist Benzotriazol. Bemerkenswerterweise zeigt die HPLC-Analyse des Produkts keine Anwesenheit des symmetrischen 5',5'-Isomeren und nur Spuren des 3',3'-isomeren Phosphonates.

Das labile Zwischenprodukt, der Phosphonigsäuretriester der allgemeinen Formel VI, wird direkt zum Phosphonat der allgemeinen Formel I oxidiert. Neben den hierfür üblichen Oxidationsmitteln wie Distickstofftetroxid oder auch Jod haben sich Peroxide, besonders wasserfreies t-Butylhydroperoxid, bewährt. Die Reaktion wird bevorzugt in einem mäßig polaren Lösemittel wie vorzugsweise Acetonitril oder Chloroform durchgeführt. Besonders zu berücksichtigen ist die bekannte (F.W. Hoffmann, R.G. Roth, T.C. Simmons, J. Amer. Chem. Soc. 80, 5937 - 40 (1958)) säurekatalysierte Umesterung der Diacylalkylphosphonite.

Die Charakterisierung der (zum Teil schon bekannten) Verbindungen erfolgt mittels $^{31}$P-NMR und $^{1}$H-NMR sowie chromatographische Vergleiche mit authentischem Material.

Die Herstellung der Verbindungen der allgemeinen Formel I, in der Z Schwefel oder Selen bedeutet, erfolgt durch direkte Umsetzung der Verbindungen der allgemeinen Formel VI mit elementarem Schwefel oder Selen. Rühren in einem polaren Lösemittel wie Tetrahydrofuran mit der stöchiometrischen Menge an Schwefel oder Selen führt in guten Ausbeuten zu den entsprechenden Thio- oder Selenophosphonaten der allgemeinen Formel I. Die Charakterisierung erfolgt neben der Elementaranalyse über $^{31}$P-NMR und $^{1}$H-NMR.

Durch die Anwesenheit eines Asymmetriezentrums am Nucleosidteil und Erzeugung eines neuen am Phosphor liegen die Phosphate der allgemeinen Formel I als Diastereomerengemisch vor (s. Tabelle 6, Isomeres 1 und 2).

Eine Variation der Parameter wie Lösemittel, Temperatur und Reihenfolge der Zugabe beeinflußt das Isomerenverhältnis nur sehr geringfügig, das nahe dem statistischen 1 : 1-Verhältnis liegt.

Die folgenden Beispiele beschreiben die Erfindung näher:

**Beispiel 1:** Ausgangsmaterial $H_3C\text{-}P[N(CH_3)_2]_2$

In einem 1000 ml-Dreihalskolben mit Tropftrichter und mechanischem Rührer werden 125 ml (1,9 Mol) Dimethylamin in 400 ml wasserfreiem Diethylether vorgelegt und unter Eiskühlung innerhalb von 60 Minuten mit 60 ml (0,40 Mol) Methyldichlorphosphan, in 200 ml wasserfreiem Ether gelöst, versetzt. Nach 2-stündigem Rühren bei Raumtemperatur und 1 Stunde bei 50°C wird unter Schutzgas der Niederschlag abfiltriert, zweimal mit 100 ml Ether nachgewaschen und das Filtrat bei etwa 0,1 bar eingeengt. Der verbleibende Rückstand wird rasch bei 0,5 bar/124°C überdestilliert. Eine Feindestillation mit einer Vigreux-Kolonne (50 cm) ergibt bei 64 - 65°C/65 mbar 36,6 g (66 % d.Th.) farblose Flüssigkeit.

Analyse: $Cl^{(-)} < 0{,}2$ %

| | |
|---|---|
| $^{31}$P-NMR (THF) | $\delta = 87$ ppm |
| $^{1}$H-NMR ($CDCl_3$) | $\delta = 1{,}23$ ppm (d, 7Hz, P-$CH_3$) |
| | $\delta = 2{,}66$ ppm (d, 7Hz, N($CH_3$)$_2$ |

**Beispiel 2:**

Die 5'-Tritylnucleoside III (1 mmol) werden in 6 ml wasserfreiem Chloroform unter inerter Stickstoffatmosphäre gelöst und dazu $H_3CP[N(\text{-}CH_3)_2]_2$ (2 mmol) gegeben. Die Reaktion ist nach 12 Stunden bei Raumtemperatur (Rühren) beendet oder bereits nach 2 Stunden durch Zugabe katalytischer Mengen (0,1 mmol) an Collidinhydrochlorid.

Anschließend wird die Lösung mit 100 ml Methylenchlorid in einen 250 ml-Scheidetrichter überführt und zweimal mit 50 ml gesättigter Kochsalzlösung (enthaltend 0,1 ml Triethylamin) ausgeschüttelt. Die organische Phase wird über wasserfreiem Natriumsulfat getrocknet und zu einem Schaum eingeengt. Dieser wird mit 50 ml Pentan 2 Stunden gerührt. Der Rückstand wird abgefiltert, in 2 ml Diethylether gelöst und langsam in gut gerührte 50 ml Pentan eingetropft. Der feine Niederschlag wird filtriert und getrocknet und ergibt 85 - 95 % Ausbeute an Verbindung der allgemeinen Formel IV (Tabellen 2 und 3).

Die Verbindungen können direkt durch $^{31}$P-Kernresonanzspektroskopie nachgewiesen werden oder nach der Oxidation mit t-Butylhydroperoxid als Phosphonsäureesteramide der allgemeinen Formel VIII (Tabellen 4 und 5).

Im $^{31}$P-NMR-Spektrum zeigen diese Substanzen bis zu 3 % an hydrolysiertem Produkt (Nucleosidmethylphosphinat), jedoch keine nachweisbare Menge an symmetrischem 3',3'-Dinucleosidphosphonit. Hierin zeigt sich die Überlegenheit gegenüber bisherigen Methoden, die stets etwa 5 - 10 % dieser Produkte lieferten. Als trockene Pulver bei -20°C aufbewahrt ist innerhalb eines Monats keine Zersetzung zu beobachten.

Analog wurden noch folgende Reagenzien eingesetzt:

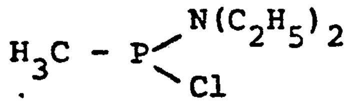

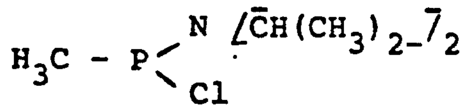

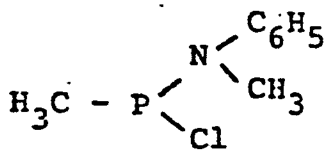

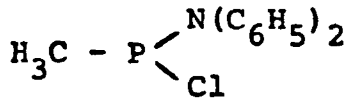

**Beispiel 3:**

Das 5'-Tritylnucleosid III (1,00 mmol) und 1,71 ml (10 mmol) N,N,N-Ethyldiisopropylamin werden in 6 ml THF vorgelegt und dann langsam 2,00 mmol Phosphonylierungsmittel II zugetropft. Nach Rühren bei Raumtemperatur über Nacht wird die Reaktionslösung in eiskaltes Wasser (50 ml, NaCl-gesättigt) eingetropft. Nach zweimaliger Extraktion mit je 20 ml Methylenchlorid trocknet man die organische Phase mit Natriumsulfat und entfernt das Lösemittel im Vakuum. Die weitere Reinigung erfolgt wie oben durch Fällen (Tabellen 2 und 3).

**Beispiel 4:**

Die Verbindung IV, 3'-0-Denzoylthymidin (0,20 mmol) und 1-H-Benzotriazol (0,80 mmol) werden in einem Rundkolben getrocknet und dann in 1,0 ml trockenem Acetonitril gelöst. Innerhalb einer Minute ist die Reaktion beendet, wobei ein sehr luft- und säurelabiles Phosphonit VI entsteht, das entweder direkt durch Oxidation mit wasserfreiem t-Butylhydroperoxid (0,25 mmol) (nach H. Langhals, E. Fritz und J. Mergelsberg, Chem. Ber. 113, 3662 (1980)), in Acetonitril oder Tetrahydrofuran gelöst, in die Phosphonate I mit 80 - 90 % Ausbeute überführt wird.

Alternativ werden zu 0,7 mmol VI bei -20°C 30 mg (0,95 mmol) Schwefel zugefügt und über Nacht bei Raumtemperatur gerührt. Meist ist die Reaktion schon nach wenigen Stunden beendet. Danach werden 20 ml Chloroform zugesetzt und die organische Phase dreimal mit Wasser ausgeschüttelt. Nach dem Trocknen über Natriumsulfat und Entfernen des Lösemittels erhält man ein Rohprodukt, das durch Kieselgelchromatographie gereinigt wird und in 80 - 90 % Ausbeute die Verbindung I ergibt (Tabelle 6).

Alternativ werden zu 0,7 mmol VI 118 mg (1,5 mmol) schwarzes Selen zugefügt und über Nacht gerührt. Nach der Aufarbeitung (wie oben) erhält man die Verbindung I in 60 % Ausbeute (Tabelle 6).

Die HPLC-Analyse des Reaktionsgemisches (im Falle: Z = 0 im Vergleich mit authentischer Referenz, P.O.P. T'so et al., Biochemistry 18, 5134 (1979)) zeigte etwa 1 % der 3',3'-Phosphonate und kein 5',5'-Isomeres.

**Tabelle 1:** Verbindungen (II) $H_3C\text{-}PXY$

| X | Y | $^{31}P$-NMR (ppm)[a)] | Kp °C/bar | $^1H$-NMR($CDCl_3$), δ (ppm) P-$CH_3$ | Sonstige |
|---|---|---|---|---|---|
| -Cl | 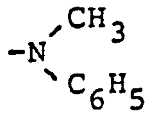 | -141,2[b)] | 44-47/$10^{-8}$ | 1,61<br>d(J = 13,1Hz) | 7,41-7,18<br>(m, 5H, Aromaten-H)<br>3,20<br>d(J = 8,3Hz) |
| -Cl | -$N(C_6H_5)_2$ | -132,2[b)] | 92-44/$10^{-8}$ | 1,53<br>d(J = 14Hz) | 7,5-6,9<br>(m, 10 H, Aromaten-H) |
| 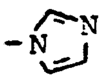 | 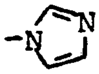 | -62[c)] | 92/$10^{-5}$<br>(Schmp: 60° C) | 2,20<br>d(J = 10Hz) | 7,45 (s, 2H)<br>6,95 (s, 4H) |
| 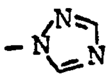 | 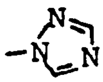 | -72[c)] | (Schmp: 110° C) | 2,32<br>d(J = 9Hz) | 8,51 (s, 2H)<br>8,07 (s, 2H) |
| 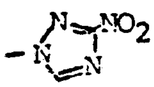 | 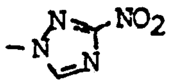 | -91[c)] | - | 2,49<br>d(J = 9Hz) | 8,89 (s, 2H) |
| 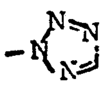 | 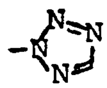 | -81[d)] | | 2,60<br>d(J = 9Hz) | 8,88 (s, 2H) |

a) rel. zu 85 % $H_3PO_4$
b) 1,2-Dichlorethan
c) THF
d) Dioxan

**Tabelle 2:** Verbindungen

(IV)

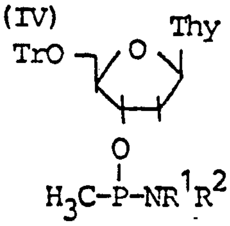

| $R^1$ | $R^2$ | $^{31}P$-NMR, δ(ppm)[a)] (1,2-Dichlorethan) | $^1H$-NMR ($CDCl_3$), δ(ppm)[b)] H-6 | $CH_3$ | P-CH | $NR^1R^2$ |
|---|---|---|---|---|---|---|
| $CH_3$ | $CH_3$ | -139,6/-140,7 | 7,60/7,57<br>(s) | 1,44/1,43<br>d(J = 1,2Hz) | 1,16/1,14<br>d(J = 7,3/7,0Hz) | 2,76/2,47<br>d(J = 8,9Hz), $N(CH_3)_2$ |
| $C_2H_5$ | $C_2H_5$ | -137,3 | 7,59/7,57<br>d(J = 1,2Hz) | 1,41/1,40<br>d(J = 1,2/1,0Hz) | 1,18/1,16<br>d(J = 7,6Hz) | 1,04/0,91<br>(d(J = 7,0 OHz), $N(C_2H_5)_2$)<br>2,76-3,09 (m, sel., komplex)<br>-$N(\text{-}CH_2CH_3)_2$ |
| $CH(CH_3)_2$ | $CH(CH_3)_2$ | -120,4 | 7,61/7,59<br>d(J = 1,2Hz) | 1,37/1,36<br>d(J = 0,9Hz) | 1,18/1,13<br>d(J = 8,5/7,9Hz) | 1,13/1,07/1,04/0,98<br>d(J = 6,7Hz), $N[CH(CH_3)_2]_2$) |
| $CH_3$ | $C_6H_5$ | -134,7/-136,2 | 7,51/7,50<br>d(J = 1,2Hz) | 1,40/1,38<br>d(J = 1,3/0,9Hz) | 1,33-1,31<br>d(J = 8,8Hz) | 3,02/2,86<br>d(J = 3,4Hz), N-$CH_3$) |
| $C_6H_5$ | $C_6H_5$ | -130,3/128,6 | 7,39/7,47<br>d(J = 1,2Hz) | 1,40/1,36<br>d(J = 1,2/0,9Hz) | 1,15/1,10<br>d(J = 9,8Hz) | |

a) relativ zu 85 % $H_3PO_4$
b) relativ zu TMS

**Tabelle 3:** Verbindungen (IV)

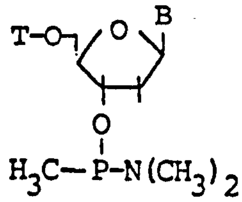

| $T^{a)}$ | $B^{b)}$ | $^{31}P$-NMR, (ppm)[c)] 1,2-Dichlorethan | $^1H$-NMR ($CDCl_3$), δ) (ppm)[d)] H-8 | H-2 | $OCH_3$ | $P-N(CH_3)_2$ | $P-CH_3$ |
|---|---|---|---|---|---|---|---|
| MMTr | $Ad^{Bz}$ | -145,3 | 8,73/8,72 (s) | 8,19/8,16 (s) | 3,76/3,75 (s) | 2,67/2,25 d(J=8,8Hz) | 1,20/1,19 d(J=7,3Hz) |
| DMTr | $C^{Bz}$ | -146,2/145,8 | | | | | |
| DMTr | $G^{iBu}$ | -144,8 | | | | | |

a) MMTr = Monomethoxy-triphenylmethyl (p-Anisoyldiphenylmethyl)
DMTr = Dimethoxy-triphenylmethyl (Di-(p-anisoyl)-phenylmethyl)
b) $Ad^{Bz}$ = 9-(N-6-Benzcyladeninyl)
$C^{Bz}$ = 1-(N-4-Benzoylcytosinyl)
$G^{iBu}$ = 9-(N-2-Isobutyroylguaninyl)
c) relativ zu 85 % $H_3PO_4$
d) relativ zu TMS

**Tabelle 4:** Verbindungen (VIII)

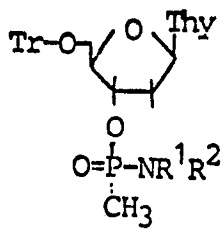

| $R^1=R^2$ | $^{31}P$-NMR, (ppm)[a)] (1,2-Dichlor-ethan) | UV ($CH_3OH$) max (log E) | $^1H$-NMR ($CDCl_3$), δ(ppm)[b)] H - 6 | P - $CH_3$ | $CH_3$ | $NR^1R^2$ |
|---|---|---|---|---|---|---|
| $CH_3$ | -40 | 263 nm (3,97) | 7,60/7,50 (d) | 1,38/1,40 d[J=16,2Hz] | 1,41/1,38 | 2,65/2,49 $N(CH_3)_2$ (d,J=9,5Hz) |
| $C_2H_5$ | -39,5[c)] | 264 nm (4,00) | 7,52 d(1,2Hz) | 1,42 d[J=16,5Hz] | 1,34 d[J=0,9Hz] | 0,94(t, J=7,1Hz) $-N(CH_2CH_3)_2$ 2,8-3,1 m, $N(C_2H_5)_2$ |
| $C_2H_5$ | -39,5[d)] | 264 nm (3,98) | 7,59 d(J=1,2Hz) | 1,38 d[J=16,5Hz] | 1,36 | 1,07(t,J=7,0Hz), $N(C_2H_5)_2$ 3,05 (dq,J=10,7Hz) J=7,0Hz, $N(C_2H_5)_2$ $CH_2CH_3$ |
| $CH(CH_3)_2$ | -38 | 264 nm (3,97) | 7,53/7,57 | 1,36 d(J=16,2Hz) | 1,31 | 1,19/1,17/1,04 (d,J=4,0/6,7/6,7Hz, $N[CH(CH_3)_2]$ |
| $C_6H_5$ | -33,5[c)] oben[e)] | 260 nm (4,09) | 7,51 (d(J=1,2Hz) | 1,68 d[J=16,8Hz] | 1,28 (s) | |

| | | | | | |
|---|---|---|---|---|---|
| $C_6H_5$ | -30,4[c) ] unten[e)] | 260 nm (4,08) | 7,48 (s) | 1,59 d[J = 217,7Hz | 1,34 s) |

a) relativ zu 85 % $H_3PO_4$
b) relativ zu TMS
c) . 1/2 $H_2O$
d) wasserfrei
e) Isomeren getrennt, relative Mobilität im DC (Ethylacetat/Methanol 100 : 4)

**Tabelle 5:** Verbindungen (VIII)

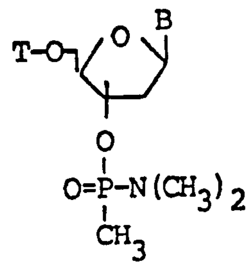

| T[a)] | B[b)] | $^{31}P$-NMR, δ (ppm)[c)] 1,2-Dichlorethan | UV ($CH_3OH$) | $^1H$-NMR ($CDCl_3$), δ (ppm) H - 8 | H - 2 | O - $CH_3$ | P-$N(CH_3)_2$ | P-$CH_3$ |
|---|---|---|---|---|---|---|---|---|
| MMTr | $Ad^{Bz}$ | -41 | 278 nm (4,39) 230 nm (4,52 | 8,73/8,68 (s) | 8,17/8,11 (s) | 3,76/3,75 (s) | 2,66/2,64 d(J = 8,9Hz) | 1,23/1,20 d(J = 7,4Hz) |
| MMTr | $C^{Bz}$ | -41,1 | | | | | | |
| DMTr | $G^{iBu}$ | -41,4 | | | | | | |

a, b), c), d): Wie bei Tabelle 3

**Tabelle 6:** Verbindungen (I)

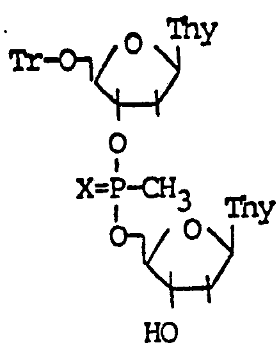

| X | $^{31}P$-NMR, δ (ppm)[a)] 1,2-Dichlorethan | UV($CH_3OH$) λ max (log E) | $^1H$-NMR, $CDCl_3$, δ (ppm)[c)] Zone[b)] | -$CH_3$ | -$CH_3$ | P-$CH_3$ |
|---|---|---|---|---|---|---|
| S | -99/97,5 | 265 nm (4,25) | oben | 1,89 (Tp) (s) | 1,43 (pT) (s) | 1,87 d(J = 15,2Hz) |
| | | | unten | 1,89 (Tp) (s) | 1,46 (pT) (s) | 1,80 d(J = 15,3Hz) |
| Se | -107,5/ -105,5 J 37 = 860Hz p = Se | 264 nm (4,26) | oben | 1,90 (Tp) d(J = 1,2Hz) | 1,42 (pT) d(J = 1,2Hz) | 2,04 d(J = 14,2Hz) |
| | | | unten | 1,98 (Tp) (s) | 1,46 (pT) (s) | 1,90 d(J = 14,5Hz) |

a) relativ zu 85 % $H_3PO_n$
b) relative Mobilität im DC (Ethylacetat/Methanol 100 : 4)
c) relativ zu TMS

**Patentansprüche** für die Vertragsstaaten BE, CH, DE, FR, GB, IT, LI, LU, NL, SE

1. Verfahren zur Herstellung von Desoxyribonukleosidphosphonaten der allgemeinen Formel I

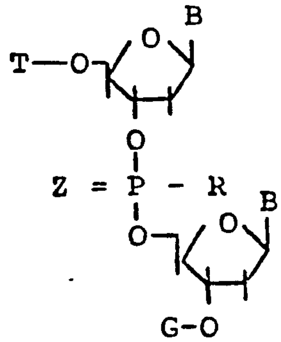

(I)

in der

T eine Schutzgruppe für eine primäre Hydroxygruppe,
B ein Nucleosidbasenrest, bei dem eine vorhandene Exo-Aminofunktion geschützt ist,
G eine Schutzgruppe für eine sekundäre Hydroxygruppe,
Z Sauerstoff, Schwefel oder Selen und
R Alkyl mit bis zu 8 C-Atomen, Cyclohexyl, Benzyl oder gegebenenfalls durch Fluor, Chlor, Brom, niederes Alkyl, niederes Alkoxy oder Trifluormethyl substituiertes Phenyl, bedeuten,

dadurch gekennzeichnet, daß man ein bifunktionelles Phosphonylierungsreagenz der allgemeinen Formel II

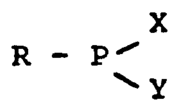

(II)

worin X Chlor oder Y und Y eine Gruppe der Formel

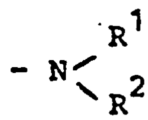

bedeuten, wobei $R^1$ und $R^2$ für gleiche oder verschiedene Alkyl- oder Cycloalkylreste mit bis zu 8 C-Atomen oder Phenylreste stehen oder $R^1$ und $R^2$ zusammen mit dem Stickstoff einen gesättigten oder ungesättigten heterocyclischen Ring, der weitere Heteroatome enthalten kann, darstellen, mit einem Nucleosid der allgemeinen Formel III

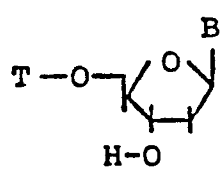

(III)

worin T und B die oben angegebene Bedeutung haben, umsetzt und die erhaltene Verbindung der allgemeinen Formel IV,

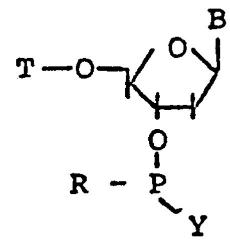

(IV)

mit einer Verbindung der allgemeinen Formel V

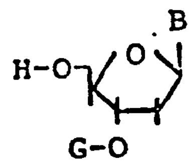

(V)

worin B und G die oben angegebene Bedeutung haben, umsetzt und die erhaltenen Verbindungen der allgemeinen Formel VI

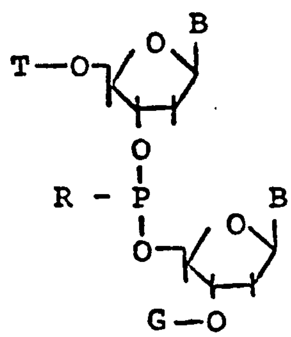

(VI)

worin T, R, B und G die oben angegebene Bedeutung haben, oxidativ in Verbindung der allgemeinen Formel I überführt.

2. Verfahren nach Anspruch 1, dadurch gekennzeichnet, daß man Verbindungen der Formeln II bis IV einsetzt, in denen

T Triphenylmethyl, p-Anisoyldiphenylmethyl oder Di-(p-anisoyl)-phenylmethyl,
B 1-Thyminyl, 1-(N-4-Benzoylcytosinyl), 9-(N-6-Benzoyladeninyl) oder 9-(N-2-Isobutyroylguaninyl) und
R Methyl, Ethyl, Phenyl oder Benzyl

bedeuten.

3. Verfahren nach Anspruch 1 oder 2, dadurch gekennzeichnet, daß man die Verbindungen der Formeln II und III bei -80 bis +100°C umsetzt.

4. Verfahren nach Anspruch 3, dadurch gekennzeichnet, daß die Umsetzung bei -20 bis 0°C stattfindet.

5. Verfahren nach einem oder mehreren der vorhergehenden Ansprüche, dadurch gekennzeichnet, daß man die Verbindungen der Formeln IV und V bei -20 bis +100°C umsetzt.

6. Verfahren nach Anspruch 5, dadurch gekennzeichnet, daß die Umsetzung bei Raumtemperatur stattfindet.

7. Verfahren nach einem oder mehreren der vorhergehenden Ansprüche, dadurch gekennzeichnet, daß man die Verbindung der Pormel VI bei -80 bis +100°C oxidativ in Verbindungen der Formel I überführt.

8. Verfahren nach Anspruch 7, dadurch gekennzeichnet, daß die Oxidation bei -20°C bis Raumtemperatur stattfindet.

9. Verbindungen der Formel IV, in der T, B, R und Y die in Anspruch 1 genannten Bedeutungen haben.

**Patentansprüche** für den Vertragsstaat AT

1. Verfahren zur Herstellung von Desoxyribonukleosidphosphonaten der allgemeinen Formel I

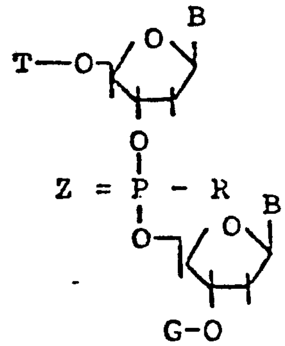

(I)

in der

T eine Schutzgruppe für eine primäre Hydroxygruppe,
B ein Nucleosidbasenrest, bei dem eine vorhandene Exo-Aminofunktion geschützt ist,
G eine Schutzgruppe für eine sekundäre Hydroxygruppe,
Z Sauerstoff, Schwefel oder Selen und
R Alkyl mit bis zu 8 C-Atomen, Cyclohexyl, Benzyl oder gegebenenfalls durch Fluor, Chlor, Brom, niederes Alkyl, niederes Alkoxy oder Trifluormethyl substituiertes Phenyl, bedeuten,

dadurch gekennzeichnet, daß man ein bifunktionelles Phosphonylierungsreagenz der allgemeinen Formel II

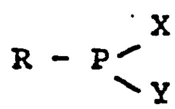

(II)

worin X Chlor oder Y und Y eine Gruppe der Formel

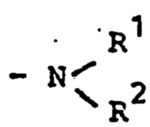

bedeuten, wobei $R^1$ und $R^2$ für gleiche oder verschiedene Alkyl- oder Cycloalkylreste mit bis zu 8 C-Atomen oder Phenylreste stehen oder $R^1$ und $R^2$ zusammen mit dem Stickstoff einen gesättigten oder ungesättigten heterocyclischen Ring, der weitere Heteroatome enthalten kann, darstellen, mit einem Nucleosid der allgemeinen Formel III

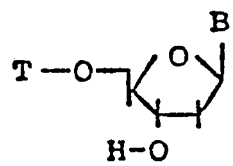

(III)

worin T und B die oben angegebene Bedeutung haben, umsetzt und die erhaltene Verbindung der allgemeinen Formel IV,

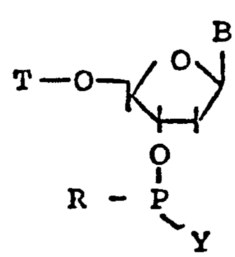

(IV)

mit einer Verbindung der allgemeinen Formel V

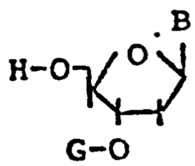

(V)

worin B und G die oben angegebene Bedeutung haben, umsetzt und die erhaltenen Verbindungen der allgemeinen Formel VI

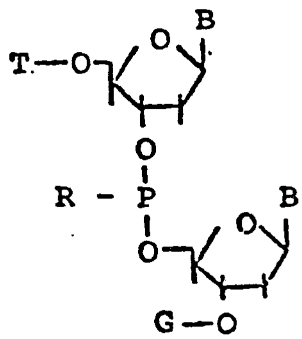

(VI)

worin T, R, B und G die oben angegebene Bedeutung haben, oxidativ in Verbindung der allgemeinen Formel I überführt.

2. Verfahren nach Anspruch 1, dadurch gekennzeichnet, daß man Verbindungen der Formeln II bis IV einsetzt, in denen

T Triphenylmethyl, p-Anisoyldiphenylmethyl oder Di-(p-anisoyl)-phenylmethyl,
B 1-Thyminyl, 1-(N-4-Benzoylcytosinyl), 9-(N-6-Benzoyladeninyl) oder 9-(N-2-Isobutyroylguaninyl) und
R Methyl, Ethyl, Phenyl oder Benzyl

bedeuten.

3. Verfahren nach Anspruch 1 oder 2, dadurch gekennzeichnet, daß man die Verbindungen der Formeln II und III bei -80 bis +100°C umsetzt.

4. Verfahren nach Anspruch 3, dadurch gekennzeichnet, daß die Umsetzung bei -20 bis 0°C stattfindet.

5. Verfahren nach einem oder mehreren der vorhergehenden Ansprüche, dadurch gekennzeichnet, daß man die Verbindungen der Formeln IV und V bei -20 bis +100°C umsetzt.

6. Verfahren nach Anspruch 5, dadurch gekennzeichnet, daß die Umsetzung bei Raumtemperatur stattfindet.

7. Verfahren nach einem oder mehreren der vorhergehenden Ansprüche, dadurch gekennzeichnet, daß man die Verbindung der Formel VI bei -80 bis +100°C oxidativ in Verbindungen der Formel I überführt.

8. Verfahren nach Anspruch 7, dadurch gekennzeichnet, daß die Oxidation bei -20°C bis Raumtemperatur stattfindet.

**Claims** for the contracting states: BE, CH, LI, DE, FR, GB, IT, LU, NL, SE

1. A process for the preparation of deoxyribonucleoside phosphonates of the general formula I

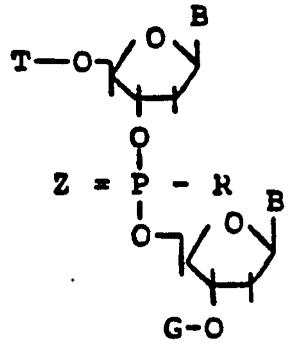

(I)

in which

T denotes a protecting group for a primary hydroxyl group,
B denotes a nucleoside base radical in which any exoamino group present is protected,
G denotes a protecting group for a secondary hydroxyl group,
Z denotes oxygen, sulfur or selenium and
R denotes alkyl having up to 8 C atoms, cyclohexyl, benzyl, or phenyl which is optionally substituted by fluorine, chlorine, bromine, lower alkyl, lower alkoxy or trifluoromethyl, characterized in that

a difunctional phosphonylating reagent of the general formula II

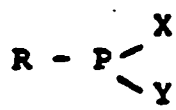

(II)

in which X denotes chlorine or Y and Y denotes a group of the formula

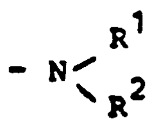

$R^1$ and $R^2$ representing identical or different alkyl or cycloalkyl radicals having up to 8 C atoms, or phenyl radicals, or $R^1$ and $R^2$, together with the nitrogen, representing a saturated or unsaturated heterocyclic ring which can contain further hetero atoms, being reacted with a nucleoside of the general formula III

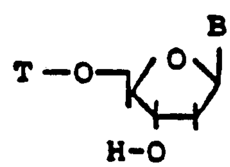

(III)

in which T and B have the meanings given above, the resulting compound of the general formula IV

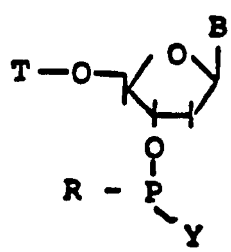

(IV)

being reacted with a compound of the general formula V

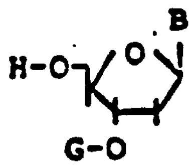

(V)

in which B and G have the meanings given above, and the resulting compounds of the general formula VI

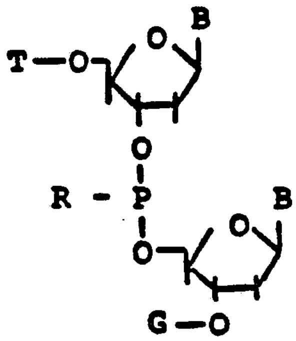

(VI)

in which T, R, B and G have the meanings given above, being oxidatively converted to compound of the general formula I.

2. The process according to claim 1, characterized in that there are used compounds of the formulae II to IV in which

T denotes triphenylmethyl, p-anisoyldiphenylmethyl or di(p-anisoyl)phenylmethyl,
B denotes 1-thyminyl, 1-(N-4-benzoylcytosinyl), 9-(N-6-benzoyladeninyl) or 9-(N-2-isobutyroylguaninyl) and
R denotes methyl, ethyl, phenyl or benzyl.

3. The process according to claim 1 or 2, characterized in that the compounds of the formulae II and III are reacted at -80 to +100°.

4. The process according to claim 3, characterized in that the reaction takes place at -20 to 0° C.

5. The process according to one or more of the preceding claims, characterized in that the compounds of the formulae IV and V are reacted at -20 to +100° C.

6. The process according to claim 5, characterized in that the reaction takes place at room temperature.

7. The process according to one or more of the preceding claims, characterized in that the compound of the formula VI is oxidatively converted to compounds of the formula I at -80 to +100° C.

8. The process according to claim 7, characterized in that the oxidation takes place at -20° C to room temperature.

9. A compound of the formula IV in which T, B, R and Y have the meanings given in claim 1.

**Claims** for the contracting state AT

1. A process for the preparation of deoxyribonucleoside phosphonates of the general formula I

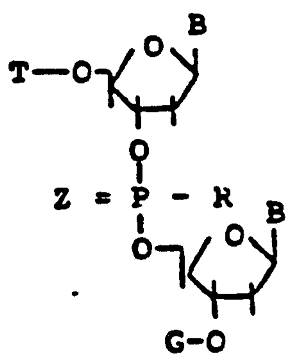

(I)

in which

T denotes a protecting group for a primary hydroxyl group,

B denotes a nucleoside base radical in which any exoamino group present is protected,
G denotes a protecting group for a secondary hydroxyl group,
Z denotes oxygen, sulfur or selenium and
R denotes alkyl having up to 8 C atoms, cyclohexyl, benzyl, or phenyl which is optionally substituted by fluorine, chlorine, bromine, lower alkyl, lower alkoxy or trifluoromethyl, characterized in that

a difunctional phosphonylating reagent of the general formula II

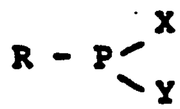

(II)

in which X denotes chlorine or Y and Y denotes a group of the formula

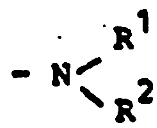

$R^1$ and $R^2$ representing identical or different alkyl or cycloalkyl radicals having up to 8 C atoms, or phenyl radicals, or $R^1$ and $R^2$, together with the nitrogen, representing a saturated or unsaturated heterocyclic ring which can contain further hetero atoms, being reacted with a nucleoside of the general formula III

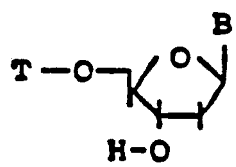

(III)

in which T and B have the meanings given above, the resulting compound of the general formula IV

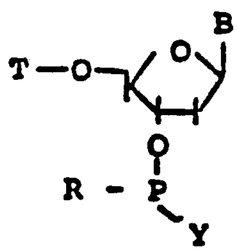

(IV)

being reacted with a compound of the general formula V

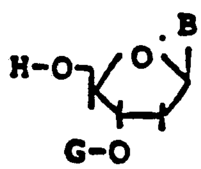

(V)

in which B and G have the meanings given above, and the resulting compounds of the general formula VI

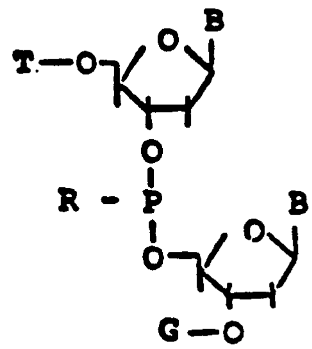

(VI)

in which T, R, B and G have the meanings given above, being oxidatively converted to compound of the general formula I.

2. The process according to claim 1, characterized in that there are used compounds of the formulae II to IV in which

T denotes triphenylmethyl, p-anisoyldiphenylmethyl or di(p-anisoyl)phenylmethyl,
B denotes 1-thyminyl, 1-(N-4-benzoylcytosinyl), 9-(N-6-benzoyladeninyl) or 9-(N-2-isobutyroylguaninyl) and
R denotes methyl, ethyl, phenyl or benzyl.

3. The process according to claim 1 or 2, characterized in that the compounds of the formulae II and III are reacted at -80 to +100° C.

4. The process according to claim 3, characterized in that the reaction takes place at -20 to 0° C.

5. The process according to one or more of the preceding claims, characterized in that the compounds of the formulae IV and V are reacted at -20 to +100° C.

6. The process according to claim 5, characterized in that the reaction takes place at room temperature.

7. The process according to one or more of the preceding claims, characterized in that the compound of the formula VI is oxidatively converted to compounds of the formula I at -80 to +100° C.

8. The process according to claim 7, characterized in that the oxidation takes place at -20° C to room temperature.

**Revendications** pour les Etats contractants: BE, CH, LI, DE, FR, GB, IT, NL, SE, LU

1. Procédé de préparation de phosphonates de désoxyribonucléosides de formule générale I

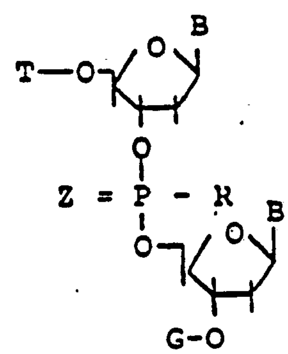

(I)

dans laquelle

T représente un groupe protecteur pour un groupe hydroxy primaire,
B représente un radical de base nucléoside dans lequel une fonction exoamino présente est protégée,
G représente un groupe protecteur pour un groupe hydroxy secondaire,
Z représente l'oxygène, le soufre ou le sélénium et
R représente un groupe alkyle ayant jusqu'à 8 atomes de carbone, un groupe cyclohexyle, benzyle ou un groupe phényle éventuellement substitué par le fluor, le chlore, le brome, un groupe alkyle inférieur, alcoxy inférieur ou trifluorométhyle,

caractérisé en ce qu'on fait réagir un réactif de phosphonylation bifonctionnel de formule générale II

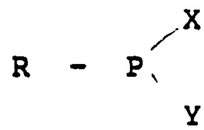

(II)

dans laquelle X représente le chlore ou Y et Y représente un groupe de formule

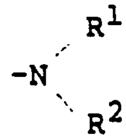

(a)

$R^1$ et $R^2$ représentant des radicaux alkyle ou cycloalkyle identiques ou différents ayant jusqu'à 8 atomes de C ou des radicaux phényle, ou $R^1$ et $R^2$ formant avec l'azote un hétérocycle saturé ou insaturé qui peut comporter d'autres hétéroatomes, avec un nucléoside de formule générale III

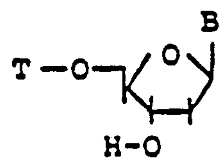

(III)

dans laquelle T et B ont la signification indiquée ci-dessus et on fait réagir le composé obtenu de formule générale IV

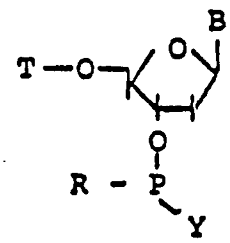

(IV)

avec un composé de formule générale V

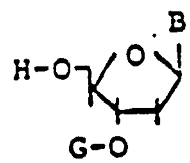

(V)

dans laquelle B et G ont la signification indiquée ci-dessus, et on convertit par oxydation les composés obtenus de formule générale VI

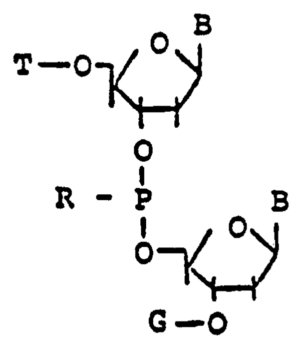

(VI)

dans laquelle T, R, B et G ont la signification indiquée ci-dessus, en composés de formule générale I.

2. Procédé selon la revendication 1, caractérisé en ce qu'on emploie des composés de formules II à IV dans lesquelles

T représente un radical triphénylméthyle, p-anisoyldiphénylméthyle ou di-(p-anisoyl)-phénylméthyle,
B représente un radical 1-thyminyle, 1-(N-4-benzoylcytosinyle), 9-(N-6-benzoyladéninyle) ou 9-(N-2-isobutyroylguaninyle)
et
R représente un radical méthyle, éthyle, phényle ou benzyle.

3. Procédé selon la renvendication 1 ou 2, caractérisé en ce que l'on fait réagir les composés de formule II et III à une température comprise entre -80 et +100° C.

4. Procédé selon la revendication 3, caractérisé en ce que la réaction a lieu à une température comprise entre -20 et 0° C.

5. Procédé selon une ou plusieurs des revendications précédentes, caractérisé en ce que l'on fait réagir les composés de formule IV et V à une température comprise entre -20 et +100° C.

6. Procédé selon la revendication 5, caractérisé en ce que la réaction a lieu à la température ambiante.

7. Procédé selon une ou plusieurs des revendications précédentes, caractérisé en ce que l'on convertit le composé de formule VI par oxydation à une température comprise entre -80 et +100° C en composé de formule I.

8. Procédé selon la revendication 7, caractérisé en ce que l'oxydation a lieu à une température comprise entre -20° C et la température ambiante.

9. Composés de formule IV dans laquelle T, B, R et Y ont les significations indiquées dans la revendication 1.

**Revendications** pour l'Etat contractant AT

1. Procédé de préparation de phosphonates de désoxyribonucléosides de formule générale I

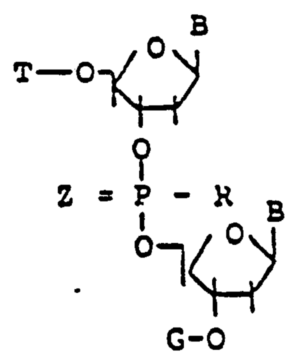

(I)

dans laquelle

T représente un groupe protecteur pour un groupe hydroxy primaire,
B représente un radical de base nucléoside dans lequel une fonction exoamino présente est protégée,
G représente un groupe protecteur pour un groupe hydroxy secondaire,
Z représente l'oxygène, le soufre ou le sélénium et
R représente un groupe alkyle ayant jusqu'à 8 atomes de carbone, un groupe cyclohexyle, benzyle ou un groupe phényle éventuellement substitué par le fluor, le chlore, le brome, un groupe alkyle inférieur, alcoxy inférieur ou trifluorométhyle,

caractérisé en ce qu'on fait réagir un réactif de phosphonylation bifonctionnel de formule générale II

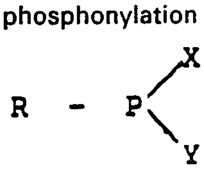

(II)

dans laquelle X représente le chlore ou Y et Y représente un groupe de formule

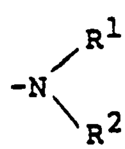

(a)

$R^1$ et $R^2$ représentant des radicaux alkyle ou cycloalkyle identiques ou différents ayant jusqu'à 8 atomes de C ou des radicaux phényle, ou $R^1$ et $R^2$ formant avec l'azote un hétérocycle saturé ou insaturé qui peut comporter d'autres hétéroatomes, avec un nucléoside de formule générale III

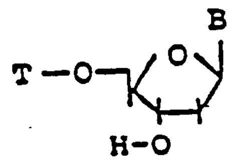

(III)

dans laquelle T et B ont la signification indiquée ci-dessus et on fait réagir le composé obtenu de formule générale IV

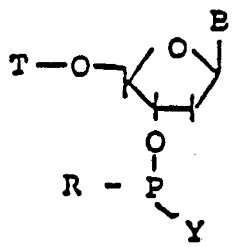

(IV)

avec un composé de formule générale V

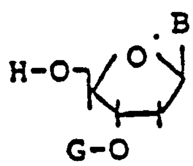

(V)

dans laquelle B et G ont la signification indiquée ci-dessus, et on convertit par oxydation les composés obtenus de formule générale VI

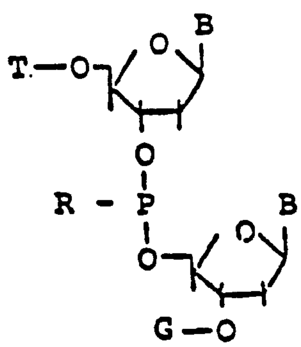

(VI)

dans laquelle T, R, B et G ont la signification indiquée ci-dessus, en composés de formule générale I.

2. Procédé selon la revendication 1, caractérisé en ce qu'on emploie des composés de formules II à IV dans lesquelles

T représente un radical triphénylméthyle, p-anisoyldiphénylméthyle ou di-(p-anisoyl)-phénylméthyle,
B représente un radical 1-thyminyle, 1-(N-4-benzoylcytosinyle), 9-(N-6-benzoyladéninyle) ou 9-(N-2-isobutyroylguaninyle)
et
R représente un radical méthyle, éthyle, phényle ou benzyle.

3. Procédé selon la renvendication 1 ou 2, caractérisé en ce que l'on fait réagir les composés de formule II et III à une température comprise entre -60 et +100° C.

4. Procédé selon la revendication 3, caractérisé en ce que la réaction a lieu à une température comprise entre -20 et 0° C.

5. Procédé selon une ou plusieurs des revendications précédentes, caractérisé en ce que l'on fait réagir les composés de formule IV et V à une température comprise entre -20 et +100° C.

6. Procédé selon la revendication 5, caractérisé en ce que la réaction a lieu à la température ambiante.

7. Procédé selon une ou plusieurs des revendications précédentes, caractérisé en ce que l'on convertit le composé de formule VI par oxydation à une température comprise entre -80 et +100° C en composé de formule I.

8. Procédé selon la revendication 7, caractérisé en ce que l'oxydation a lieu à une température comprise entre -20° C et la température ambiante.